# EUROPEAN PATENT APPLICATION

(11) **EP 1 050 315 A2**
(43) Date of publication of application: **08.11.2000**
(21) Application number: 00109486.1
(22) Date of filing: 04.05.2000
(51) Int. Cl.: A61M 25/00

(54) **Drainage tube**

(30) Priority: 07.05.1999 IT TO990077
(71) Applicant: Karbix Establishment, 9490 Vaduz (LI)
(72) Inventor: Ugo, Ferrando, 10128 Torino (IT)
(74) Representative: Lotti, Giorgio

(57) **Abstract**

Drainage tube (1) presenting a main duct (4), a distal drainage portion (5) arranged at a free end of the duct (4) itself and an intrinsic blocking device for blocking the tube (1) itself; the intrinsic blocking device is defined by an inflatable balloon (13) which is arranged along the duct (4) above the distal portion (5) and is suitable for being inflated near an entry opening (2) of the drainage tube (1) into the body of the patient; and a fixing flap (14) integral to the duct (4) and arranged on opposite sides of the distal portion (5) with regard to the balloon (13) which is used to further fix the tube (1) with regard to the entry opening(2).

## Description

The present invention relates to a drainage tube for use in medical applications.

Normally, drainage tubes of a well-known type are inserted into a patient's body via an entry opening, and comprise a main duct presenting a distal drainage portion suitable for being inserted via the opening itself, and an outlet portion suitable for remaining outside the body of the patient.

Once the distal portion has been inserted via the entry opening, it is necessary to block the drainage tube in relation to the opening itself in order to avoid any accidental movement of the tube itself which might prejudice its correct function.

European Patent Application no. EP O 356 683 presents a blocking device for drainage tubes and similar devices which comprises a rigid fixing plate, a bridge-shaped closing element integral to the plate and suitable for engaging the drainage tube, and two holes obtained through the plate itself on opposite sides of the bridge-shaped element.

The blocking device described in the above-mentioned patent application has a number of disadvantages which are essentially due to the dimensions of the device itself, as well as to the difficulties inherent in installing it and the time taken to carry out the installation. In fact, as a matter of normal practice, medical personnel usually resort to the use of adhesive plasters which are directly attached in contact with the outlet portion and the skin of the patient.

In any case, neither the use of the above-mentioned blocking device, nor the use of the above-mentioned adhesive plaster, can guarantee that the drainage tube is fixed in the best possible way.

The aim of the present invention is to realise a drainage tube which will resolve the above-mentioned disadvantages in a cost-effective manner and which will also be simple and quick to install.

According to the present invention, a drainage tube will be realised comprising a main duct, a distal drainage portion arranged at a free end of the duct itself and suitable for being inserted into the body of a patient via an entry opening; the tube being characterised by the fact that it comprises an intrinsic blocking device for blocking the duct in relation to the said entry opening, and comprising an internal one-way internal blocking portion arranged along the duct above the distal portion and suitable for being arranged near the entry opening in order to prevent the tube from being accidentally extracted.

The invention will now be described with reference to the attached drawings, which illustrate a non-limiting embodiment of the invention, in which:
FIGURE 1 shows a side elevated view of a preferred form of embodiment of the drainage tube according to the present invention; and
FIGURE 2 shows a second along the line II-II of FIGURE 1.

With reference to the attached drawings, the number 1 is used to indicate a drainage tube, in its entirety, for draining fluids from the body of a patient suitable for being inserted into the patient's body itself via an entry opening 2 obtained in the skin 3 of the patient him/herself.

The tube 1 comprises a main duct 4, a distal drainage portion 5 arranged at an open end of the duct 4 itself, and an intrinsic blocking device which is an integral part of the tube 1 itself, and is suitable for anchoring the tube 1 to the patient's body in such a way as to prevent any axial movement whatsoever of the tube 1 in relation to the entry opening 2.

The tube 1 comprises, finally, a safety valve 7 arranged substantially in correspondence with an end portion 8 of the duct 4 in order to isolate the tube 1 completely from the outside each time that it is necessary to replace a fluid collection bag (of a well-known type, not illustrated) which is connected to the tube 1 itself.

The distal portion 5 comprises an opening 9 shaped like a flute mouthpiece, and a number of holes 10 at the same distance from each other along a longitudinal axis A of the duct 4, and arranged in two diametrically opposite rows.

The blocking device 6 is a two-way blocking device and comprises an internal one-way blocking portion 11, which is arranged along the duct 4 above the distal portion 5, and is suitable for being arranged near the entry opening 2 in order to prevent the tube 12 from being accidentally removed. The device 6 also comprises an external one-way blocking portion 12 arranged along the duct 4 on opposite sides of the distal portion 5 in relation to the internal portion 11 in order to prevent the tube 1 from being accidentally introduced.

The internal portion 11 comprises an inflatable balloon 13 which is arranged along the duct 4 and is provided with a linking duct 13a so that it can be inflated near the entry opening 2 in order to prevent any axial movement of the tube 1 towards the outside of the entry opening 2 itself.

The external portion 12 comprises a fixing flap 14 integral to the duct 4 and arranged on opposite sides of the distal portion 5 in relation to the balloon 13 in order to further fix the tube 1 in relation to the entry opening 2 and prevent any axial movement of the tube 1 towards the inside of the entry opening 2 itself.

The flap 14 is rigidly fixed to the duct 4 on the outside of the duct 4 itself and at a determined distance from the balloon 13, and comprises a central opening 15, and an annular portion 16 which extends around the opening 15 itself.

The external portion 12 comprises an annular plate 17, either together with the flap 14 or on its own instead of the flap 14 itself, the annular plate 17 being arranged along the duct 4 near the skin 3 of the patient and provided with a central hole 18 which is slidingly engaged by the duct 4, and a fixing ring 19 which can be associated to the plate 17 in order to fix the plate 17 itself to the duct 4 itself.

In the case in which both the flap 14 and the plate 17 are present at the same time, they should be arranged in sequence along the duct 4 with the flap 14 being completely covered by the plate 17. As an alternative, the flap 14 could be directly assembled onto the plate 17 in such a way as to correspond to the border of the plate 17 itself.

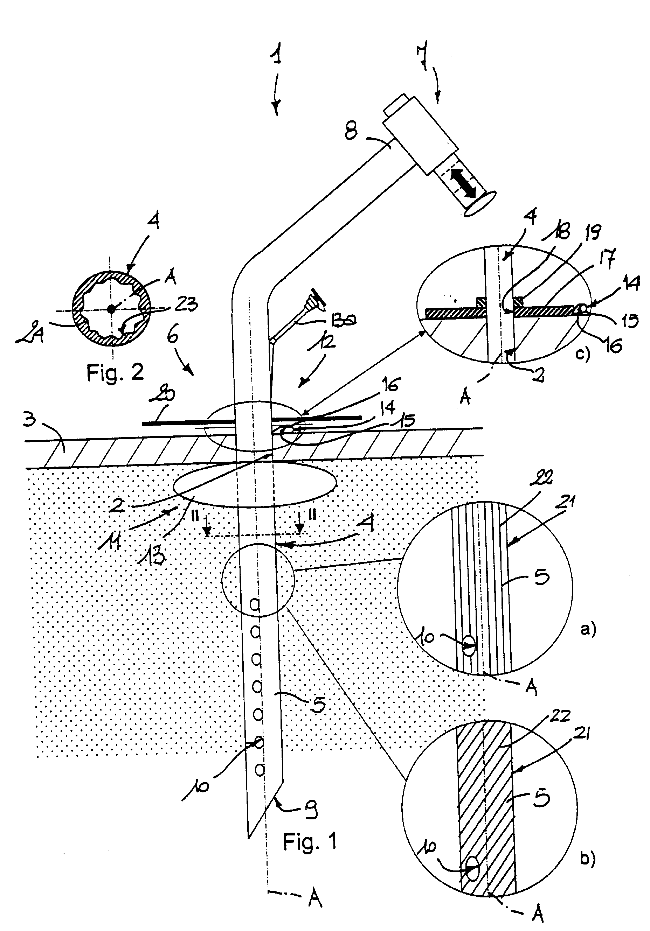

The device 6, finally, comprises a pre-medicated pad 20 arranged along the duct 4 substantially to correspond to the flap 14, and suitable for acting as a barrier to prevent any eventual infections in the vicinity of the entry opening 2. In Figure 1, the pad 20 is shown in a position prior to being used, otherwise, when in use, it is arranged close to the entry opening 2 and the flap 14.

According to the illustration shown in Figure 1 and in the enlargements "a" and "b", the duct 4 presents an external surface 21, which can either be smooth or provided with a number of projections 22 obtained on the outside of the duct 4 itself and arranged either parallel to each other and to the axis A or, alternatively, according to respective helixes with a substantially constant pitch.

According to the illustration shown in Figure 2, the duct 4 presents an internal surface 23, which can either be smooth or provided with a number of projections 24 obtained on the inside of the duct 4 itself and arranged either parallel to each other and to the axis A or, alternatively, according to respective helixes with a substantially constant pitch.

When in use, the tube 1 is selected in function of the length L of the respective distal portion 5, and is inserted into the patient's body via the entry opening 2 in such a way that the opening 9 in the shape of a flute mouthpiece reaches the required position. At this point, the balloon 13 is inflated and it adheres to the subcutaneous tissue so that any accidental movement is prevented, as is any axial movement of the tube 1 towards the outside, and an internal barrier is also created in order to prevent any micro-organisms escaping towards the outside of the entry opening 2.

The relative position of the balloon 13 to the flap 14 along the duct 4 is such that, once the balloon 13 has been inflated, the flap 14 is immediately arranged outside the entry opening 2 and substantially in contact with the patient's skin. In this way, with a simple suture of the kind normally in use in medical practice, it is possible to engage the annular portion 16 of the flap 14 in such a way as to perfectly anchor the flap 14 itself to the patient's skin.

Once the suture has been effected it is possible to apply the pre-medicated pad by means of a simple operation.

As has previously been described, the plate 17 may be used as an alternative to the flap 14, the plate 17 being made to slide along the duct 4 in such a way that it is arranged in contact with the skin 3 and then blocked by the ring 19. In the case in which the flap 14 is required in conjunction with the plate 17, it is possible to block the flap 14 and the plate 17 one after the other using the methods previously described, or the plate 17 may be used with the flap 14 fixed to the pointed border of the flap 14 itself.

It is obvious from what has been previously described that the presence of the flap 14 or the plate 17 means that any operations necessary to block the tube 1 to the patient's body are rendered both rapid and simple and that it is possible to avoid any crushing of the duct 4 as well as the use of any fixing devices which have to be applied to the patient's body itself.

It is intended that the invention should not be limited to the forms of embodiment herein described and illustrated, which are to be considered purely as examples of the drainage tube herein described, and it should be made clear that said drainage tube could be subject to further modifications in terms of its shape, the arrangement of its parts and the details relating to its construction and assembly.

## Claims

1. Drainage tube (1) comprising a main duct (4), a distal drainage portion (5) arranged at a free end of the duct (4) itself and suitable for being inserted into the body of a patient via an entry opening (2); the tube (1) being characterised by the fact that it comprises an intrinsic blocking device for blocking the duct (4) in relation to the said entry opening, and comprising an internal one-way internal blocking portion (11) arranged along the duct (4) above the distal portion (5), and suitable for being arranged near the entry opening (2) in order to prevent the tube (1) from being accidentally extracted.

2. Tube according to Claim 1, characterised by the fact that the said internal blocking portion (11) comprises as inflatable balloon (13) which is suitable for being inflated near the opening (2).

3. Tube according to Claims 1 or claim 2, characterised by the fact that the said intrinsic blocking device (6) comprises an external one-way blocking portion (12) arranged on opposite sides of the distal portion (5) in relation to said internal blocking portion (11) in order to further fix the tube (1) in relation to the entry opening (2).

4. Tube according to Claim 3, characterised by the fact that the said external blocking portion (12) comprises an annular plate (17) provided with a central hole (18) which is slidingly engaged by the duct (4), and a fixing ring (19) which can be associated to the plate (17) in order to fix the plate (17) itself to the duct (4).

5. Tube according to Claim 3, characterised by the fact that the said external blocking portion comprises a fixing flap (13) integral to the duct (4).

6. Tube according to Claim 5, characterised by the fact that the said flap (13) comprises a central opening (15) and annular portion (16) which extends around the central portion (15) itself.

7. Tube according to Claim 4, Claim 5 or Claim 6, characterised by the fact that the said annular plate (17) and the said fixing flap (13) are arranged in sequence along the main duct (4).

8. Tube according to Claim 4, Claim 5 or Claim 6, characterised by the fact that the said fixing flap (13) is integrally assembled to the said annular plate (17).

9. Tube according to any of the previous Claims, characterised by the fact that the said duct (4) presents a longitudinal axis (A) and a number of projections (21) which are obtained on the outside of the duct (4) itself along the longitudinal axis (A) itself.

10. Tube according to Claim 9, characterised by the fact that the said projections (21) are parallel to each other and to said longitudinal axis (A).

11. Tube according to Claim 9, characterised by the fact that the said projections (21) are bent in relation to said longitudinal axis (A).

12. Tube according to any of the previous Claims, characterised by the fact that the said duct (4) presents a longitudinal axis (A) and a number of projections (24) which are obtained on the inside of the duct (4) itself along the longitudinal axis (A) itself.

13. Tube according to Claim 12, characterised by the fact that the said projections (24) are parallel to each other and to the said longitudinal axis (A).

14. Tube according to Claim 12, characterised by the fact that the said projections (24) are bent in relation to said longitudinal axis (A).

15. Tube according to any of the previous Claims, characterised by the fact of comprising valve means (7) for separating arranged at a second open end (7) of the duct (4) in order to completely separate the duct (4) itself from the surrounding environment itself.
